# EUROPEAN PATENT APPLICATION

(11) **EP 3 309 551 A1**
(43) Date of publication of application: **18.04.2018**
(21) Application number: 16807886.3
(22) Date of filing: 16.06.2016
(51) Int. Cl.: G01N 33/574, C12Q 1/68, C12N 15/115, A61K 31/517, A61K 31/7088, A61K 39/395

(54) **NOVEL BIOMARKER FOR DIAGNOSING RESISTANCE TO ANTICANCER AGENT FOR BILIARY TRACT CANCER AND USE THEREOF**

(30) Priority: 12.06.2015 KR 20150083578; 30.05.2016 KR 20160066719
(71) Applicant: Cowell Biodigm Co., Ltd., Seodaemun-gu Seoul 03722 (KR)
(72) Inventor: SONG, Si Young, Seoul 04194 (KR); PARK, Soo Bin, Seoul 03938 (KR)
(74) Representative: Zardi, Marco
(86) International application number: PCT/KR2016/006421
(87) International publication number: WO 2016/200246

(57) **Abstract**

The present invention provides a novel molecule marker for resistance to an anticancer agent for biliary tract cancer, and diagnostic and therapeutic uses thereof. The marker, according to the present invention, shows significantly increased expression in an anticancer agent-resistant biliary tract cancer cell line compared to a non-anticancer agent-resistant biliary tract cancer cell line. By inhibiting the expression of protein in the marker according to the present invention, sensitivity of anticancer agent-resistant biliary tract cancer to the anticancer agent can be increased.

<110>     INDUSTRY-ACADEMIC COOPERATION FOUNDATION, Yonsei University

<120>     Novel Biomarker for Diagnosis of Anticancer drug Resistance of
          Biliary tract Cancer and Uses thereof

<130>     PP160059

<150>     KR 15/0083578
<151>     2015-06-12

<150>     KR 16/0066719
<151>     2016-05-30

<160>     32

<170>     KopatentIn 2.0

<210>     1
<211>     22
<212>     DNA
<213>     Artificial Sequence

<220>
<223>     forward primer for Notch3


<400>     1
atggtgggaa ctaaacacag ct                                                22


<210>     2
<211>     20
<212>     DNA
<213>     Artificial Sequence

<220>
<223>     reverse primer for Notch3


<400>     2
atgaccctgg aggaagcaca                                                   20


<210>     3
<211>     20
<212>     DNA
<213>     Artificial Sequence

<220>
<223>     forward primer for Hes1


<400>     3
gtgctgtctg gatgcggagt                                                   20


<210>     4
<211>     21
<212>     DNA
<213>     Artificial Sequence

<220>
<223>     reverse primer for Hes1

<400>    4
gaacactcac actcaaagcc c                                                21


<210>    5
<211>    20
<212>    DNA
<213>    Artificial Sequence

<220>
<223>    forward primer for Ihh


<400>    5
cctgaactcg ctggctatct                                                  20


<210>    6
<211>    20
<212>    DNA
<213>    Artificial Sequence

<220>
<223>    reverse primer for Ihh


<400>    6
aatacaccca gtcaaagccg                                                  20


<210>    7
<211>    20
<212>    DNA
<213>    Artificial Sequence

<220>
<223>    forward primer for Gli1


<400>    7
agagtccagg gggttacata                                                  20


<210>    8
<211>    20
<212>    DNA
<213>    Artificial Sequence

<220>
<223>    reverse primer for Gli1


<400>    8
agagtccagg gggttacata                                                  20


<210>    9
<211>    22
<212>    DNA
<213>    Artificial Sequence

<220>
<223>    forward primer for Nanog


<400>    9
actgtctctc ctcttccttc ct                                                22


<210>    10
<211>    22
<212>    DNA
<213>    Artificial Sequence

<220>
<223>    reverse primer for Nanog


<400>    10
agagtaaagg ctggggtagg ta                                                22


<210>    11
<211>    20
<212>    DNA
<213>    Artificial Sequence

<220>
<223>    forward primer for Oct4


<400>    11
gtggaggaag ctgacaacaa                                                   20


<210>    12
<211>    20
<212>    DNA
<213>    Artificial Sequence

<220>
<223>    reverse primer for Oct4


<400>    12
agcagcctca aaatcctctc                                                   20


<210>    13
<211>    20
<212>    DNA
<213>    Artificial Sequence

<220>
<223>    forward primer for PTEN


<400>    13
ggacgaactg gtgtaatgat                                                   20


<210>    14
<211>    20
<212>    DNA

<213>    Artificial Sequence

<220>
<223>    reverse primer for PTEN


<400>    14
cagaccacaa actgaggatt                                                    20


<210>    15
<211>    20
<212>    DNA
<213>    Artificial Sequence

<220>
<223>    forward primer for FZD7


<400>    15
ccaacggcct gatgtacttt                                                    20


<210>    16
<211>    20
<212>    DNA
<213>    Artificial Sequence

<220>
<223>    reverse primer for FZD7


<400>    16
gccatgccga agaagtagag                                                    20


<210>    17
<211>    22
<212>    DNA
<213>    Artificial Sequence

<220>
<223>    forward primer for beta-catenin


<400>    17
gtatgagtgg gaacagggat tt                                                 22


<210>    18
<211>    20
<212>    DNA
<213>    Artificial Sequence

<220>
<223>    reverse primer for beta-catenin


<400>    18
cctggtcctc gtcatttagc                                                    20


<210>    19

<211>    20
<212>    DNA
<213>    Artificial Sequence

<220>
<223>    forward primer for C-met


<400>    19
caatgtgaga tgtctccagc                                                    20


<210>    20
<211>    20
<212>    DNA
<213>    Artificial Sequence

<220>
<223>    reverse primer for C-met


<400>    20
ccttgtagat tgcaggcaga                                                    20


<210>    21
<211>    38
<212>    DNA
<213>    Artificial Sequence

<220>
<223>    forward primer for Snail


<400>    21
aagcttccat ggcgcgctct ttcctcgtca ggaagccc                                38


<210>    22
<211>    39
<212>    DNA
<213>    Artificial Sequence

<220>
<223>    reverse primer for Snail


<400>    22
ggatcctcag cggggacatc ctgagcagcc ggactcttg                               39


<210>    23
<211>    20
<212>    DNA
<213>    Artificial Sequence

<220>
<223>    forward primer for Vimentin


<400>    23
gagaactttg ccgttgaagc                                                    20

<210>    24
<211>    20
<212>    DNA
<213>    Artificial Sequence

<220>
<223>    reverse primer for Vimentin


<400>    24
gcttcctgta ggtggcaatc                                                    20


<210>    25
<211>    20
<212>    DNA
<213>    Artificial Sequence

<220>
<223>    forward primer for CD90


<400>    25
gacccgtgag acaaagaagc                                                    20


<210>    26
<211>    20
<212>    DNA
<213>    Artificial Sequence

<220>
<223>    reverse primer for CD90


<400>    26
actgtgacgt tctgggagga                                                    20


<210>    27
<211>    20
<212>    DNA
<213>    Artificial Sequence

<220>
<223>    forward primer for CEACAM1


<400>    27
atacctgcca cgccaataac                                                    20


<210>    28
<211>    20
<212>    DNA
<213>    Artificial Sequence

<220>
<223>    reverse primer for CEACAM1


<400>    28

ttatgctgag ggtggtgttg                                                    20

<210>    29
<211>    20
<212>    DNA
<213>    Artificial Sequence

<220>
<223>    forward primer for CD151

<400>    29
ctccccggac atactctctg                                                    20

<210>    30
<211>    20
<212>    DNA
<213>    Artificial Sequence

<220>
<223>    reverse primer for CD151

<400>    30
gtcagagctc acctggcttc                                                    20

<210>    31
<211>    20
<212>    DNA
<213>    Artificial Sequence

<220>
<223>    forward primer for beta-actin

<400>    31
ggcatcctca ccctgaagta                                                    20

<210>    32
<211>    20
<212>    DNA
<213>    Artificial Sequence

<220>
<223>    reverse primer for beta-actin

<400>    32
ggggtgttga aggtctcaaa                                                    20

## Description

### [Technical Field]

The present invention was undertaken under the project No. HI14C1324 with the support of the Korean Ministry of Health and Welfare, the research management organization for this project is the Korean Health Industry Development Institute, the title of the research enterprise is "R&D for Developing Research-driven Hospitals," the title of the research project is "Construction of Open Access Business Platform for Strategic Technology Integration to Meet Global Healthcare Demand," the supervision institution is the Office of Research Affairs/Industry-Academic Cooperation Foundation of Yonsei University, and the research period is October 01, 2014 to March 31, 2023.

This application claims priority to and the benefit of Korean Patent Application No. 10-2015-0083578, filed on June 12, 2015 and Korean Patent Application No. 10-2016-0066719, filed on May 30, 2016, the disclosures of which are incorporated herein by reference in their entireties.

The present invention relates to a novel biomarker associated with resistance to an anticancer agent for biliary tract cancer and a diagnostic and therapeutic use thereof.

### [Background Art]

Bile ducts are a group of tube-like structures that send bile produced in the liver to the duodenum and gradually converge in the liver and become thicker like tree branches gathering together toward one branch, and when coming from the liver, left and right bile ducts are usually combined as a single duct. Bile ducts are classified into intrahepatic bile ducts passing through the liver and extrahepatic bile ducts extending to the duodenum out of the liver. In the extrahepatic bile ducts, a pouch that temporarily stores bile for concentration is called a gallbladder, and a collection of the intrahepatic and extrahepatic bile ducts and the gallbladder is called a biliary tract.

Biliary tract cancer is also called cholangiocarcinoma, which is a malignant tumor occurs in epithelium of the bile duct. Biliary tract cancer is a type of incurable cancer.70 to 80% of this cancer is diagnosed as advanced cancer, 30 to 40% of this cancer can be treated by surgery, and its 5-year survival rate is only 7%.

Although a variety of anticancer agents for various types of cancer have been developed so far, few types of cancer can be cured using solely anticancer agents. This is because cancer cells may not respond to an anticancer agent upon cancer treatment using an anticancer agent, or while a tumor size is effectively reduced in the early stage, cancer cells may become resistant to an anticancer agent during or after treatment. Therefore, for effective anticancer treatment, forms of resistance to an anticancer agent such as cancer cells' tolerance to an anticancer agent should be overcome.

Resistance to an anticancer agent becomes an issue in many cases even in an early stage of biliary tract cancer, and thus the anticancer agent response rate is only 15%, and the recurrence rate after surgery approaches 85%. Even so, there are almost no effective anticancer agents that can be used in adjuvant chemotherapy before and after surgery. Accordingly, there is a demand for a novel biomarker that can find the occurrence of anticancer agent resistance at an early stage of a therapeutic process for biliary tract cancer and for the development of a novel anticancer agent that can overcome resistance to an anticancer agent.

It has been reported that transgelin-2 (TAGLN2) protein is a human protein encoded by the *TAGLN2* gene, and expression of this protein is increased in colorectal cancer (Prior Art Document 1) and proportional to a degree of bladder cancer progression (Prior Art Document 2). However, the relationship between TAGLN2 and resistance to an anticancer agent for biliary tract cancer has not been reported yet.

Throughout this specification, various papers and patent documents are provided as references and cited references thereof are represented. The disclosure of the cited theses and patent literatures are incorporated herein by reference in its entirety, and thus the level of the field of art including the present application and the scope of the present application are more fully described.

### [Prior Art Documents]

### [Non-specific documents]

Cancer Sci February 2010 vol. 101, No. 2, 523-29 (published on November 23, 2009)
British Journal of Cancer, 3/1/2011, Vol. 104 Issue 5, p808 (published on March in 2001)

### [Disclosure]

### [Technical Problem]

The inventors attempted to find a novel biomarker that is suitable for exact molecular diagnosis of resistance to an anticancer agent for biliary tract cancer in an early stage, and carried out research for developing therapeutic agents to overcome resistance to an anticancer agent for biliary tract cancer. As a result, they confirmed that TAGLN2 expression is significantly increased in an anticancer agent-resistant biliary tract cancer cell line than in an anticancer agent-non-resistant biliary tract cancer cell line, verified that the TAGLN2 protein is involved in acquisition of anticancer agent resistance, and further confirmed that the sensitivity (susceptibility) to an anticancer agent of anticancer agent-resistant biliary tract cancer can be enhanced by inhibiting TAGLN2 expression, and therefore the present invention was accomplished.

Therefore, the present invention is directed to providing a kit for diagnosing resistance to an anticancer agent for biliary tract cancer.

The present invention is also directed to providing a kit for detecting a marker for resistance to an anticancer agent for biliary tract cancer.

The present invention is also directed to providing a method for detecting a marker for resistance to an anticancer agent for biliary tract cancer.

The present invention is also directed to providing a pharmaceutical composition for inhibiting resistance to an anticancer agent for biliary tract cancer.

Other objects and advantages of the present invention will become more apparent from the following detailed description, claims, and drawings of the present invention.

### [Technical Solution]

In one aspect of the present invention, the present invention provides a kit for diagnosing resistance to an anticancer agent for biliary tract cancer, which includes an antibody, aptamer, oligopeptide, or peptide nucleic acid (PNA) which binds to TAGLN2 protein or includes a primer or probe binding to a nucleic acid molecule encoding the protein.

In another aspect of the present invention, the present invention provides a kit for detecting a marker for resistance to an anticancer agent for biliary tract cancer, which includes an antibody, aptamer, oligopeptide, or PNA which binds to TAGLN2 protein, or includes a primer or probe binding to a nucleic acid molecule encoding the protein.

The inventors attempted to find a novel biomarker that is suitable for exact molecular diagnosis of resistance to an anticancer agent for biliary tract cancer at in an early stage, and carried out research for developing therapeutic agents to overcome the resistance to an anticancer agent for biliary tract cancer. As a result, they confirmed that TAGLN2 expression is significantly higher in an anticancer agent-resistant biliary tract cancer cell line than in an anticancer agent-non-resistant biliary tract cancer cell line, verified that the TAGLN2 protein is involved in acquisition of anticancer agent resistance, and further confirmed that susceptibility to an anticancer agent for anticancer agent-resistant biliary tract cancer can be enhanced by inhibiting the TAGLN2 expression.

The expression "kit for diagnosing resistance to an anticancer agent for biliary tract cancer" used herein refers to a kit including a "composition for diagnosing resistance to an anticancer agent for biliary tract cancer." Accordingly, the expression "kit for diagnosing resistance to an anticancer agent for biliary tract cancer" used herein can be used alternately or together with a "composition for diagnosing resistance to an anticancer agent for biliary tract cancer."

The term "diagnosis" used herein includes determining susceptibility to an anticancer agent of biliary tract cancer, determining whether the occurred biliary tract cancer has resistance to an anticancer agent at a present time, or prognosing anticancer agent-resistant biliary tract cancer (e.g., determining the responsibility of the cancer to anticancer treatment).

The inventors confirmed that whether resistance to an anticancer agent for biliary tract cancer is acquired can be rapidly and exactly diagnosed from biological samples taken from a subject by means of the marker of the present invention.

The term "diagnostic marker, marker for diagnosis or diagnosis marker" used herein refers to a substance that can identify anticancer agent-resistant biliary tract cancer cells, and to an organic biomolecule indicating an increase in biliary tract cancer cells acquiring resistance to an anticancer agent. For the object of the present invention, the marker for diagnosing resistance to an anticancer agent for biliary tract cancer includes TAGLN2 protein and a nucleic acid molecule (DNA or mRNA) encoding the same.

According to an exemplary embodiment of the present invention, the kit of the present invention is an immunoassay kit, that is, a kit that can confirm the presence and expression level of TAGLN2 protein by using an immunoassay method (antigen-antibody reaction). For example, the kit is a Luminex assay kit, protein microarray kit, or ELISA kit.

In one exemplary embodiment, the kit of the present invention includes an antibody specifically binding to TAGLN2 protein to measure the presence and expression level of TAGLN2 protein.

In the present invention, the antibody refers to a specific protein molecule designated to an antigenic site. For the object of the present invention, the antibody refers to an antibody specifically binding to a marker protein, and includes all of polyclonal antibodies, monoclonal antibodies, and recombinant antibodies.

As described above, a novel marker protein for diagnosing resistance to an anticancer agent for biliary tract cancer was defined, and thus production of an antibody using the marker protein can be easily performed using a technique widely known in the art. For example, polyclonal antibodies may be produced by a widely known method for collecting serum containing antibodies such as injecting the marker protein antigen into an animal and collecting blood from an animal. Such polyclonal antibodies can be prepared from a host derived from any animal species such as goat, rabbit, sheep, monkey, horse, pig, cattle, or dog.

Monoclonal antibodies may be prepared using a method well known in the art such as a hybridoma method (refers to Kohler and Milstein (1976) European Journal of Immunology 6:511-519), or phage antibody libraries (Clackson et al, Nature, 352:624-628, 1991; Marks et al, J. Mol. Biol., 222:58, 1-597, 1991). The antibody prepared by the above-described method can be isolated and purified using a method such as gel electrophoresis, dialysis, salt precipitation, ion exchange chromatography, affinity chromatography, etc.

The antibody of the present invention includes a functional fragment of an antibody molecule, as well as a complete form having two full-length light chains and two full-length heavy chains. The functional fragment of the antibody molecule refers to a fragment having at least an antigen-binding function, for example, Fab, F(ab'), F(ab')2, or Fv.

The kit of the present invention may be used to diagnose resistance to an anticancer agent for biliary tract cancer in a conventional immunoassay method. Such immunoassay may be performed according to various quantitative or qualitative immunoassay protocols which have been conventionally developed.

The immunoassay formats include radioimmunoassay, radioimmunoprecipitation, immunoprecipitation, immunohistochemistry, enzyme-linked immunosorbent assay (ELISA), capture-ELISA, inhibition or competition assay, sandwich assay, flow cytometry, immunofluorescent staining, and immunoaffinity purification, but the present invention is not limited thereto. A method for immunoassay or immunostaining is disclosed in Enzyme Immunoassay, E. T. Maggio, ed., CRC Press, Boca Raton, Florida, 1980; Gaastra, W., Enzyme-linked immunosorbent assay (ELISA), in Methods in Molecular Biology, Vol. 1, Walker, J.M. ed., Humana Press, NJ, 1984; and in Ed Harlow and David Lane, Using Antibodies: A Laboratory Manual, Cold Spring Harbor Laboratory Press, 1999, the disclosures of which are incorporated herein by reference in their entireties.

For example, when the method of the present invention is performed according to radioimmunoassay, a radioisotope (e.g., C¹⁴, I¹²⁵, P³² or S³⁵)-labeled antibody may be used to detect a marker molecule of the present invention.

For example, when the method of the present invention is performed according to ELISA, a specific exemplary embodiment of the present invention includes (a) coating the surface of a solid substrate with an unknown cell sample lysate or cell culture to be analyzed; (b) reacting the cell lysate or cell culture with an antibody against a marker as a primary antibody; (c) reacting the resulting product of Step (b) with an enzyme-linked secondary antibody; and (d) measuring activity of the enzyme.

The enzyme linked to the secondary antibody includes an enzyme that catalyzes a color reaction, a fluorescence reaction, a luminescence reaction, or an infrared ray reaction, but the present invention is not limited thereto, and for example includes alkaline phosphatase, β-galactosidase, horseradish peroxidase, luciferase, and cytochrome P450. When the alkaline phosphatase is used as the enzyme linked to the secondary antibody, a color reaction substrate such as bromochloroindolyl phosphate (BCIP), nitro blue tetrazolium (NBT), naphthol-AS-Bl-phosphate or enhanced chemifluorescence (ECF) may be used, and when the horse radish peroxidase is used, a substrate such as chloronaphthol, minoethylcarbazol, diaminobenzidine, D-luciferin, bis-N-methylacridinium nitrate (lucigenin), resorufin benzyl ether, luminol, 10-acetyl-3,7-dihydroxyphenoxazine (amflex red), p-phenylenediamine-HCl and pyrocatechol (HYR), tetramethylbenzidine (TMB), 2,2'-Azine-di[3-ethylbenzthiazoline sulfonate] (ABTS), o-phenylenediamine (OPD) and naphthol/pyronine, glucose oxidase, nitroblue tetrazolium (t-NBT), or phenzaine methosulfate (m-PMS) may be used.

In immunoassay methods including ELISA, measuring activity of the final enzyme or measuring a signal may be performed according to various methods known in the art. Such detection of a signal allows qualitative or quantitative analysis of the marker of the present invention. A signal may be easily detected with streptavidin when biotin is used as a label, or with luciferin when luciferase is used as a label.

On the other hand, the kit of the present invention may be used in western blotting using one or more antibodies against a marker protein. All proteins are isolated from a sample, separated by size through electrophoresis, transferred to a nitrocellulose membrane, and reacted with antibodies. An amount of proteins produced by gene expression is confirmed by a method for confirming an amount of the produced antigen-antibody complex with labeled antibodies, and thus resistance to an anticancer agent for biliary tract cancer may be confirmed.

Alternatively, the kit of the present invention may be used in immunohistochemistry using one or more antibodies against the marker protein.

In the kit of the present invention, instead of an antibody, an aptamer specifically binding to the marker protein of the present invention may be included. The aptamer is an oligonucleotide or peptide molecule, and general information on the aptamer is described in detail in Bock LC et al., Nature 355(6360):5646(1992); Hoppe-Seyler F, Butz K "Peptide aptamers: powerful new tools for molecular medicine," and J Mol Med. 78(8):42630(2000); Cohen BA, Colas P, Brent R. "An artificial cell-cycle inhibitor isolated from a combinatorial library" Proc Natl Acad Sci USA. 95(24): 142727(1998).

The kit of the present invention may further include other components, in addition to the above-mentioned component. For example, when the kit of the present invention is applied to PCR amplification, the kit of the present invention may selectively include reagents necessary for PCR amplification such as a buffer solution, a DNA polymerase (e.g., a thermally stable DNA polymerase obtained from Thermus aquaticus (Taq), Thermus thermophilus (Tth), Thermus filiformis, Thermis flavus, Thermococcus literalis or Pyrococcus furiosus (Pfu)), a DNA polymerase cofactor, and dNTPs. The kit of the present invention may be manufactured by a plurality of separate packages or compartments including the above-mentioned reagent components.

According to an exemplary embodiment of the present invention, the kit of the present invention is a microarray or gene amplification kit. When the kit of the present invention is a microarray, a probe is fixed onto a solid surface of the microarray, and when the kit of the present invention is a gene amplification kit, the kit includes a primer.

The probe or primer included in the diagnosis kit of the present invention has a complementary sequence with respect to a TAGLN2 nucleotide sequence. The term "complementary" used herein refers to complementarity to an extent of being selectively hybridized with the above-mentioned nucleotide sequence under a certain specific hybridization or annealing condition. Therefore, the term "complementary" used herein has a different meaning from being perfectly complementary, and the primer or probe of the present invention may have one or more mismatch base sequences in order to be selectively hybridized with a nucleotide sequence.

The term "primer" used herein refers to a single-stranded oligonucleotide that can act as the start point of template-directed DNA synthesis at a suitable condition in a suitable buffer solution under suitable conditions (that is, four types of different nucleoside triphosphate and polymerase). A suitable length of the primer is changed according to various factors, for example, a temperature and a use of the primer, but typically is 15 to 30 nucleotides. The design of such a primer may be easily performed by those of ordinary skill in the art with reference to the nucleotide sequence of TAGLN2 using, for example, a primer design program (e.g., PRIMER 3 program).

The term "probe" used herein refers to a linear oligomer of natural or modified monomers or linkages, and may include a deoxyribonucleotide and a ribonucleotide, be specifically hybridized with a target nucleotide sequence, and exist in nature or be artificially synthesized.

A nucleotide sequence of the marker of the present invention which should be referred to in order to manufacture a primer or probe may be identified from GenBank, and with reference to this sequence, the primer or probe may be designed.

In the microarray of the present invention, the probe is used as a hybridizable array element and fixed onto a substrate.

Sample DNA or mRNA applied to the microarray of the present invention may be labeled and hybridized with an array factor on the microarray. Hybridization conditions may vary. Detection and analysis of hybridization may be performed in various ways according to a labeling substance.

A label for the probe may provide a signal for detecting hybridization and may be linked to the oligonucleotide. Suitable labels include fluorophores (e.g., fluorescein, phycoerythrin, rhodamine, lissamine, and Cy3 and Cy5 (Pharmacia)), chromophores, chemiluminescents, magnetic particles, radioactive isotopes (P³² and S³⁵), mass labels, electron-dense particles, enzymes (alkaline phosphatase or horseradish peroxidase), cofactors, substrates for enzymes, heavy metals (e.g., gold), and heptenes having a specific binding partner such as an antibody, streptavidin, biotin, deoxygenin, or a chelating group, but the present invention is not limited thereto. The labeling may be performed using various methods conventionally used in the art, for example, a nick translation method, a random priming method (Multiprime DNA labelling systems booklet, "Amersham" (1989)), and a carnation method (Maxam & Gilbert, Methods in Enzymology, 65:499(1986)). The label provides a signal that can be detected using fluorescence, radiation, coloring measurement, weight measurement, X-ray diffraction or absorption, magnetism, enzymatic activity, mass analysis, binding affinity, high frequency for hybridization, or nanocrystals.

Nucleic acid samples subjected to analysis may be prepared using mRNA obtained from various biosamples. The biosamples are, for example, biliary tract cancer cells or a culture thereof, biliary tract cancer tissue, blood, serum and plasma. Hybridization-based analysis may be performed even by labeling with cDNA for analysis, instead of a probe.

The term "amplification" used while describing the "gene amplification kit" refers to a reaction in amplifying a nucleic acid molecule. Various amplification reactions such as a polymerase chain reaction (RT-PCR; Sambrook et al., Molecular Cloning. A Laboratory Manual, 3rd ed. Cold Spring Harbor Press(2001)), methods of Miller, H. I. (WO 89/06700) and Davey, C. et al. (EP 329,822), a ligase chain reaction (LCR; 17, 18), Gap-LCR (WO 90/01069), repair chain reaction (EP 439,182), transcription-mediated amplification (TMA; WO 88/10315), self-sustained sequence replication (WO 90/06995), selective amplification of target polynucleotide sequences (US Patent No. 6,410,276), consensus sequence primed polymerase chain reaction (CP-PCR; US Patent No. 4,437,975), arbitrarily primed polymerase chain reaction (AP-PCR; US Patent Nos. 5,413,909 and 5,861,245), nucleic acid sequence based amplification (NASBA; US Patent Nos. 5,130,238, 5,409,818, 5,554,517, and 6,063,603), and strand displacement amplification and loop-mediated isothermal amplification (LAMP) have been known in the art.

In still another aspect of the present invention, the present invention provides a method for detecting a marker for resistance to an anticancer agent for biliary tract cancer by detecting TAGLN2 protein contained in a biological sample or in a nucleotide molecule encoding the protein in order to provide information necessary for diagnosis of resistance to an anticancer agent for biliary tract cancer.

Since the method for detecting a marker for resistance to an anticancer agent for biliary tract cancer and the kit for diagnosing resistance to an anticancer agent for biliary tract cancer utilize the same marker, to avoid excessive complexity of the specification, redundant information of both will not be repeated.

According to an exemplary embodiment of the present invention, the method is performed by the above-described antigen-antibody reaction or gene amplification method. Expression levels may be compared, using the detection methods, between a marker protein of a control or a nucleic acid molecule encoding the same and a marker protein of a biliary tract cancer patient-derived biological sample intended for analysis or a nucleic acid molecule encoding the same, and the resistance to an anticancer agent for biliary tract cancer may be diagnosed by determining whether there is a significant change in the expression level.

As described above, the present invention determines the resistance to an anticancer agent for biliary tract cancer using a molecular diagnosis method. The marker of the present invention is a biomolecule existing at a high concentration in anticancer agent-resistant biliary tract cancer. The term "high concentration" use herein refers to a high amount of a marker in a biological sample to be examined in comparison to a control sample. For example, as a result of analysis using the above-described analysis method, when the marker of the present invention is detected at an amount 2 to 10 times larger than that of the control, it is determined as being of "high concentration" in the present invention and as exhibiting resistance to an anticancer agent for biliary tract cancer. Thus, it can be confirmed whether the sample has resistance to an anticancer agent for biliary tract cancer.

In the scope of the control sample, biliary tract cancer patient-derived cells identified as not having acquired resistance to an anticancer agent, a culture and tissue thereof, blood, serum, and plasma are also included.

The biological sample for analysis may also be selected from the group consisting of biliary tract cancer cells or a culture thereof, biliary tract cancer tissue, blood, serum, and plasma.

In yet another aspect of the present invention, the present invention provides a pharmaceutical composition for inhibiting resistance to an anticancer agent for biliary tract cancer, which includes a TAGLN2 activation inhibitor or expression inhibitor as an active ingredient.

According to an exemplary embodiment of the present invention, the TAGLN2 expression inhibitor of the present invention includes one or more selected from the group consisting of an antisense oligonucleotide complementarily binding to mRNA of a TAGLN2 gene or TAGLN2 expression-promoting gene, small interfering RNA (siRNA), small hairpin RNA (shRNA), and ribozyme, and the TAGLN2 activation inhibitor includes one or more selected from the group consisting of a an antibody binding to TAGLN2 and a fragment, a compound, a peptide, a peptide mimetic and an aptamer, which bind to an antigen thereof.

The TAGLN2 activation inhibitor or expression inhibitor used in the present invention inhibits resistance to an anticancer agent for biliary tract cancer. The term "inhibition of resistance to an anticancer agent" has the same meaning as "anticancer sensitization" or "chemical sensitization," and means that, when an anticancer agent includes a composition for inhibiting anticancer agent resistance, rather than including no composition, toxicity of the anticancer agent toward biliary tract cancer is further reinforced or increased in the treatment of cancer, resistance to the anticancer agent is reduced, and the anticancer agent works more effectively. In the present invention, the composition including a TAGLN2 inhibitor sensitizes biliary tract cancer cells toward an anticancer agent, resistance of biliary tract cancer cells to an anticancer agent may be reduced, and an effect of the anticancer agent may be increased.

According to an exemplary embodiment of the present invention, the anticancer agent is selected from the group consisting of gemcitabine, 5-fluorourasil (5-FU), oxaliplatin, carboplatin, and cisplatin.

The antisense oligonucleotide is a DNA or RNA sequence that can bind to TAGLN2 mRNA, and may inhibit activities necessary for translation, translocation into the cytoplasm, maturation or other overall biological functions of TAGLN2 mRNA. The length of the antisense nucleic acid is 6 to 100 bp, preferably 8 to 60 bp, and more preferably 10 to 40 bp.

The antisense oligonucleotide may be modified at one or more base, sugar, or backbone sites to enhance effectiveness (De Mesmaeker et al., Curr Opin Struct Biol., 5(3):343-55(1995)).

The antisense oligonucleotide may be synthesized *in vitro* according to a conventional method for administration into a living body, or may be synthesized *in vivo.*

The design of the antisense oligonucleotide that can be used in the present invention may be easily manufactured according to a method known in the art (Weiss, B. (ed.): Antisense Oligodeoxynucleotides and Antisense RNA: Novel Pharmacological and Therapeutic Agents, CRC Press, Boca Raton, FL, 1997; Weiss, B., et al., Antisense RNA gene therapy for studying and modulating biological processes. Cell. Mol. Life Sci., 55:334-358(1999).

According to an exemplary embodiment of the present invention, the TAGLN2 expression inhibitor of the present invention is shRNA or siRNA including a sequence complementary to the TAGLN2 gene.

The term "small hairpin RNA or short hairpin RNA (shRNA)" used herein refers to an RNA sequence making a firm hairpin turn, which may be used to silence gene expression through RNA interface.

The term "small interference RNA (siRNA)" used herein refers to a nucleic acid molecule capable of mediating RNA interference or gene silencing (refer to WO 00/44895, WO 01/36646, WO 99/32619, WO 01/29058, WO 99/07409 and WO 00/44914). Since siRNA can inhibit the expression of a target gene, it facilitates an efficient gene knock-down method or gene therapy.

The siRNA molecule of the present invention may have a double-helix structure in which a sense strand is located opposite an antisense strand. In addition, the siRNA molecule of the present invention may have a single helix structure having self-complementary sense and antisense strands. The term "complementary" used herein encompasses being 100% complementary and also being incompletely complementary.

An activation inhibitor of the TAGLN2 protein may be an antibody specifically binding to the TAGLN2 protein, or a fragment, compound, peptide, peptide mimetic or aptamer, which binds to an antigen thereof. The peptide specifically binding to the TAGLN2 protein may be obtained using a typical method known in the art, for example a phage displaying method (Smith GP, "Filamentous fusion phage: novel expression vectors that display cloned antigens on the virion surface." Science 228 (4705):13151317(1985); Smith GP, Petrenko VA, "Phage display." Chem. Rev. 97(2):391410(1997)). The peptide mimetic inhibits a TAGLN2 protein-binding domain, thereby inhibiting activity of the TAGLN2 protein. The peptide mimetic may be a peptide or non-peptide, which may consist of amino acids connected by non-peptide binding such as psi binding (Benkirane, N., et al. J.Biol. Chem., 271:33218-33224, 1996).

The pharmaceutical composition of the present invention may be orally or parenterally (e.g., intravenously, subcutaneously, intraperitoneally or locally) administered according to a desired method, and a dosage may vary according to a patient's body weight, age, sex, health condition and diet, and administration time, administration method, duration or intervals of administration, excretion rate, constitutional peculiarity, or preparation characteristics.

The pharmaceutical composition of the present invention may be formulated in various preparations for administration, and may be formulated in various forms by adding excipients. The excipients are pharmaceutically suitable all types of non-toxic and inactive solid, quasi-solid or liquid formulation supplements, and may include, for example, a filler, a thickening agent, a binder, a wetting agent, a disintegrating agent, a dispersing agent, a surfactant or a diluting agent.

### [Advantageous Effects]

Characteristics and advantages of the present invention are summarized as follows:
(a) The present invention provides a novel molecular marker for resistance to an anticancer agent for biliary tract cancer and a diagnostic and therapeutic use thereof.
(b) The marker of the present invention is considerably higher in expression in ananticancer agent-resistant biliary tract cancer cell line than in an anticancer agent-non-resistant biliary tract cancer cell line.
(c) Susceptibility of anticancer-resistant biliary tract cancer to the anticancer agent may be improved by inhibiting expression of the marker protein of the present invention.

### [Description of Drawings]

FIG. 1a is the result of an MTT assay showing that IC₅₀ for an anticancer agent is higher in an anticancer agent-resistant biliary tract cancer cell line (SNU1196/GR) than in a parent cell line.
FIG. 1b is the result of western blotting showing that TAGLN2 expression and secretion are increased in an anticancer agent-resistant biliary tract cancer cell line (SNU1196/GR).
FIGS. 2 to 7 show that CD151 is a biliary tract cancer stem cell marker, but is reduced in an anticancer agent-resistant biliary tract cancer cell line:
FIG. 2a shows sphere cells having characteristics of cancer stem cells cultured from human biliary tract cancer cell lines;
FIG. 2b is the results of RT-PCR showing overexpression of cancer stem cell-related genes in sphere cells of a biliary tract cancer cell line;
FIG. 2c is the results of western blotting showing overexpression of a cancer stem cell marker c-MET-associated signaling system and epithelial-to-mesenchymal transition-associated proteins in the cultured sphere cells isolated from the biliary tract cancer cell line;
FIG. 3a is the result of a clonogenic assay showing the characteristic of the sphere cells cultured in the biliary tract cancer cell line;
FIG. 3b shows a tumor size after 12 weeks of subcutaneous injection of each of adherent cells and sphere cells of the biliary tract cancer cell line into nude mice;
FIG. 4a is the result of western blotting showing that CD151 is overexpressed in the biliary tract cancer sphere cells having the characteristic of the cancer stem cells;
FIG. 4b is the results of RT-PCR showing CD151 expression in 6 types of human biliary tract cancer cell lines;
FIG. 4c is the results of immunohistochemistry showing overexpression of a biliary tract cancer stem cell marker CD151 in human biliary tract cancer tissue;
FIG. 5a is the result of sorting cells after indirect FACS staining of the human biliary tract cancer cell line using a CD151 antibody;
FIG. 5b is the results of RT-PCR showing that cancer stem cell-related genes are increased in cells overexpressing CD151 in cells isolated by the CD151 antibody;
FIG. 5c shows that cell migration ability is increased in cells overexpressing CD151 among the cells isolated by the CD151 antibody;
FIG. 5d is the result of a clonogenic assay showing that clonogenic ability, which is a characteristic of cancer stem cells, is increased in the CD151-overexpressing cells among the cells isolated by the CD 151 antibody;
FIG. 6a is the result of RT-PCR showing reduction of CD151 expression over time after transfection of the human biliary tract cancer cell line with CD151 siRNA;
FIG. 6b is the result of western blotting showing change in various signaling systems associated with cancer stem cells according to inhibition of CD151 expression;
FIG. 6c shows that cancer cell migration ability is reduced when CD151 expression is inhibited in the human biliary tract cancer cell line;
FIG. 6d is the result of sphere forming assay showing that self-renewal ability, which is the most important characteristic, of caner stem cells when a human biliary tract cancer cell line is transfected with CD151 siRNA; and
FIG. 7 is the result of western blotting showing that the biliary tract cancer stem cell marker CD151 is reduced in an anticancer agent-resistant biliary tract cancer cell line.
FIG. 8a is the result of western blotting showing that TAGLN2 expression is inhibited in an anticancer agent-resistant biliary tract cancer cell line by siRNA transfection.
FIG. 8b is the result of an MTT assay showing that susceptibility to gemcitabine is increased when TAGLN2 expression is inhibited in an anticancer agent-resistant biliary tract cancer cell line.
FIG. 8c is the result of an MTT assay showing that susceptibility to 5-FU is increased when TAGLN2 expression is inhibited in an anticancer agent-resistant biliary tract cancer cell line.
FIG. 8d is the result of an MTT assay showing that susceptibilities to platinum-like anticancer agents such as cisplatin, oxaliplatin, and carboplatin are increased when TAGLN2 expression is inhibited in an anticancer agent-resistant biliary tract cancer cell line.
FIG. 8e is the result of an MTT assay showing that there are no changes in susceptibilities to topoisomerase inhibitors such as ironotecan and etoposide, and an EGFR inhibitor such as erlotinib when TAGLN2 expression is inhibited in an anticancer agent-resistant biliary tract cancer cell line.

### [Modes of the Invention]

Hereinafter, the present application will be described in further detail with reference to examples. The examples are merely provided to more fully describe the present application, and it will be obvious to those of ordinary skill in the art that the scope of the present application is not limited to the following examples.

### Examples

### Experimental methods

### Construction of anticancer agent-resistant biliary tract cancer cell line

To create an anticancer agent-resistant biliary tract cancer cell line, a SNU1196 cell line was treated with gemcitabine at various concentrations and then subjected to MTT assay to calculate an IC₅₀ value, and the cells were treated with gemcitabine at a concentration of IC₅₀ for 3 days. After 3 days, when the cells were recovered after being cultured in a drug-free growth medium [10% fetal bovine serum (FBS; Hyclone)-containing RPMI1640 (Invitrogen Gibco)], the cells were recovered by treating with gemcitabine at double the IC₅₀ concentration for 3 days, and an anticancer agent-resistant cell line (named SNU1196/GR) was established by repeating such a cell culturing procedure for 1 year. Whether the resistance to the anticancer agent was acquired was confirmed by MTT assay.

### Confirmation of resistance to anticancer agent by MTT assay

Cells derived from biliary tract cancer cell line SNU1196 and anticancer agent-resistant biliary tract cancer cell line SNU1196/GR were attached to 96-well plates at 3 × 10³ cells/well, and the following day, the cells were cultured for 72 hours by treating with gemcitabine at various concentrations. After 72 hours, an MTT (3-[4,5-dimethylthiazol-2-yl]-2,5 diphenyl tetrazolium bromide, Sigma-Aldrich) solution was added to the cells at 100 µl/well, and was allowed to react for 3 hours while protected from light. After 3 hours, the MTT solution was removed, and then absorbance was measured at 570 nm by adding dimethyl sulfoxide (DMSO) at 100 µl/well.

### Western blotting

Cellular proteins were obtained by treating cells with accutase (Sigma-Aldrich), and then washed with PBS and extracted using a cell lysis buffer [70 mM β-glycerophosphate, 0.6 mM Na vanadate, 2 mM MgCl₂, 1 mM EGTA, 1 mM DTT, 0.5% Triton X100, 0.2 mM PMSF, and a protease inhibitor cocktail (Roche)]. For extraction of secretion proteins, cells were cultured in a serum-free RPMI medium for 48 hours, and then cell debris was removed using centrifugation and a filter to collect a supernatant. Secretion proteins in the supernatant were concentrated by acetone precipitation, and then extracted using a cell lysis buffer. Protein quantification was performed through Bradford assay (Bio-rad), and proteins were loaded into an SDS-polyacrylamide gel at the same concentration, followed by electrophoresis. The proteins fractionated by size through electrophoresis were transferred to a PVDF membrane (Millipore), and then blocked in 5% non-fat milk-added TBS-T (Tris-buffered saline/0.05% Tween-20) for 1 hour to reduce non-specific reactions. Afterward, each membrane was cultured overnight with a primary antibody at 4 □. As the primary antibody, rabbit polyclonal TAGLN2 (Sigma-Aldrich), mouse monoclonal GAPDH, mouse monoclonal β-catenin, rabbit monoclonal occludin, mouse monoclonal CD151, rabbit polyclonal E-cadherin (Santa Cruz Biotechnology), rabbit polyclonal phospho-AKT, rabbit polyclonal phosphor-ERK, rabbit polyclonal Snail, rabbit polyclonal phospho-GSK3, rabbit polyclonal Oct4 (Cell Signaling Technology), or mouse monoclonal N-cadherin (Abcam plc) was used. Each membrane reacting with the primary antibody was washed with a TBS-T buffer, and reacted with an HRP-conjugated secondary antibody (Santa Cruz Biotechnology) for 1 hour. Each protein was detected using an enhanced chemiluminescence system (PIERCE).

### Evaluation of overcoming anticancer agent resistance by inhibiting TAGLN2 expression in anticancer agent-resistant biliary tract cancer cell line

As siRNA for TAGLN2, sc-106633 produced by Santa Cruz Biotechnology was used, and as control siRNA, sc-37007 (Santa Cruz Biotechnology) was used. An anticancer agent (gemcitabine)-resistant biliary tract cancer cell line SNU1196/GR was transfected with each of control siRNA and TAGLN2 siRNA using a lipofectamine RNAiMAX transfection reagent (Invitrogen), and after 24 hours, was cultured for 72 hours by treating with gemcitabine at various concentrations. The cell line reacted with an MTT solution (Sigma-Aldrich) for 3 hours, and then absorbance at 570 nm was measured by treating with DMSO to determine whether the anticancer agent resistance was overcome through inhibition of TAGLN2 expression in the anticancer agent-resistant biliary tract cancer cell line. The inhibition of the expression by TAGLN2 siRNA was confirmed by western blotting after 72 hours of the transfection.

### Separation and culturing of sphere having characteristic of cancer cell line

For sphere culturing, cells were seeded at 1000 cell/ml into a 6-well ultra low-attachment plate (Corning) containing a serum-free RPMI medium supplemented with EGF (10 ng/ml, R&D Systems), bFGF (10 ng/ml, R&D Systems), 1× insulin transferrin selenium (ITS, Gibco) and 0.5% BSA (Invitrogen Gibco), and were cultured for 7 to 10 days. Cells grown in an adherent state while attached to a culture dish (Nalgene) with the same culture medium were used as a control.

### Analysis of expression of cancer stem cell-related genes through semi-quantitative RT-PCR

Total RNA was extracted from cells using an RNAeasy Mini kit (QIAGEN). 2 µg of each extracted RNA was reverse-transcripted through a Superscript II (GibcoBRL) reaction at 42 □ for one hour. Expression of cancer stem cell-related genes were analyzed by PCR using primers for cancer stem cell-related genes and a Taq polymerase, and expression of beta-actin genes was used as a control. Primer sequences for the cancer stem cell-related genes are listed in Table 1.

**[Table 1]**

| Gene | Forward primer (SEQ ID NO:) | Reverse primer (SEQ ID NO:) |
|---|---|---|
| Notch3 | atggtgggaa ctaaacacag ct (1) | atgaccctgg aggaagcaca (2) |
| Hes1 | gtgctgtctg gatgcggagt (3) | gaacactcac actcaaagcc c (4) |
| Ihh | cctgaactcg ctggctatct (5) | aatacaccca gtcaaagccg (6) |
| Gli1 | agagtccagg gggttacata (7) | agagtccagg gggttacata (8) |
| Nanog | actgtctctc ctcttccttc ct (9) | agagtaaagg ctggggtagg ta (10) |
| Oct4 | gtggaggaag ctgacaacaa (11) | agcagcctca aaatcctctc (12) |
| PTEN | ggacgaactg gtgtaatgat (13) | cagaccacaa actgaggatt (14) |
| FZD7 | ccaacggcct gatgtacttt (15) | gccatgccga agaagtagag (16) |
| β-catenin | gtatgagtgg gaacagggat tt (17) | cctggtcctc gtcatttagc (18) |
| C-met | caatgtgaga tgtctccagc (19) | ccttgtagat tgcaggcaga (20) |
| Snail | aagcttccat ggcgcgctct ttcctcgtca ggaagccc (21) | ggatcctcag cggggacatc ctgagcagcc ggactcttg (22) |
| Vimentin | gagaactttg ccgttgaagc (23) | gcttcctgta ggtggcaatc (24) |
| CD90 | gacccgtgag acaaagaagc (25) | actgtgacgt tctgggagga (26) |
| CEACAM1 | atacctgcca cgccaataac (27) | ttatgctgag ggtggtgttg (28) |
| CD151 | ctccccggac atactctctg (29) | gtcagagctc acctggcttc (30) |
| β-actin | ggcatcctca ccctgaagta (31) | ggggtgttga aggtctcaaa (32) |

### Analysis of expression of biliary tract cancer stem cell-related protein CD151 in human biliary tract cancer tissue through immunohistochemistry

A human biliary tract cancer tissue slide was deparaffinated in xylene, and rehydrated in alcohol by grade. Activity of an endogenous peroxidase was blocked with 0.3% hydrogen peroxide-containing methanol solution at room temperature for 20 minutes. Microwave antigen retrieval was carried out in a sodium citrate buffer (0.01 M, pH 6.0) for 5 minutes. After that, a slide was cultured in a 10% normal donkey serum solution for 1 hour to reduce non-specific background staining. A blocked section was cultured overnight with a mouse monoclonal CD151 (Santa Cruz Biotechnology) antibody diluted at 1:500 at 4 □. A subsequent reaction was carried out according to the standard protocol included in an Envision kit (DakoCytomation). Finally, the slide was cultured with 3,3'-diaminobenzidine (DakoCytomation), and counter-stained with a Harris hematoxylin (Sigma-Aldrich) solution.

### Verification of self-renewal and colony formation through soft agar colony forming assay

Soft agar colony forming assay was carried out by modifying the standard protocol (MJ Son et al., Cell stem cells: 4, 440-452(2009)). Difco agar noble (Becton Dickinson) was used, and 0.6% bottom agar was added to a 24-well plate with a 2× sphere culture medium at a ratio of 1:1 and solidified at room temperature. A 0.3% top agar was prepared by mixing cells suspended in a sphere culture medium and agar, and then plated on the bottom agar. The sphere culture medium containing a growth factor was replaced every 2 or 3 days. For 2 to 3 weeks, the cells were cultured in an incubator at 37 □ with CO₂ and stained with a crystal violet solution, and then cell colonies were counted, followed by comparative analyses of self-renewal and anchorage-independent growth.

### Confirmation of carcinogenesis of sphere cells isolated and cultured in biliary tract cancer cell line SNU1196

Adherent and sphere cells cultured from human biliary tract cancer cell line SNU1196 were treated with 0.25% trypsin EDTA, thereby preparing suspensions of the same number of single cells (1,000 cells). Subsequently, the cell suspension was subcutaneously injected into both sides of a nude mouse (Orient Bio Inc.). Afterward, the mouse was monitored for 12 weeks, and dissected to confirm carcinogenic ability of the sphere cells.

### Cell isolation using biliary tract cancer stem cell-related marker CD151 antibody, and confirmation of characteristic of cancer stem cells of isolated cells

Suspensions of the same number of single cells were prepared by treating SNU1196 cells with accutase. Indirect staining was carried out using a mouse monoclonal CD151 antibody (Abcam) or a control normal mouse IgG (Santa Cruz Biotechnology) on ice for 20 minutes. Afterward, the resulting cells were treated with a PE goat anti-mouse IgG (BD Biosciences PharMingen) secondary antibody and cultured on ice for 20 minutes. Cell sorting was carried out using FACSAria II (BD Immunocytochemistry System). Semi-quantitative RT-PCR, cancer cell migration analysis and soft agar colony forming assay were performed on sorted cells to analyze the characteristics of cancer stem cells.

### Cancer cell migration assay

Cancer cell migration assay was carried out on a transwell (Corning). 85 µl of matrigel (BD Biosciences PharMingen) diluted in serum-free media at a ratio of 1:4 was added to an upper well and solidified at 37 □. 1.0 × 10⁴ cells suspended in serum-free media were seeded into an upper well, 500 µl of an NIH3T3 fibroblast culture medium was seeded into a lower well, and then culturing proceeded for 24 hours. The cells attached under a filter after passing through the filter were fixed and stained, and then the number of migrated cells was measured by microscopy.

### Inhibition of expression by transfection of CD151 siRNA

siRNA for CD151 was sc-42829 (Santa Cruz Biotechnology). Transfection was carried out using a Lipofectamine RNAiMAX transfection reagent (Invitrogen), and after 30, 72, and 96 hours, inhibition of CD151 expression was confirmed by RT-PCR or western blotting. As a control, cells transfected with control siRNA (Santa Cruz, sc-3 7007) were used.

### Example 1. Confirmation of TAGLN2 overexpression in anticancer agent-resistant biliary tract cancer stem cell line

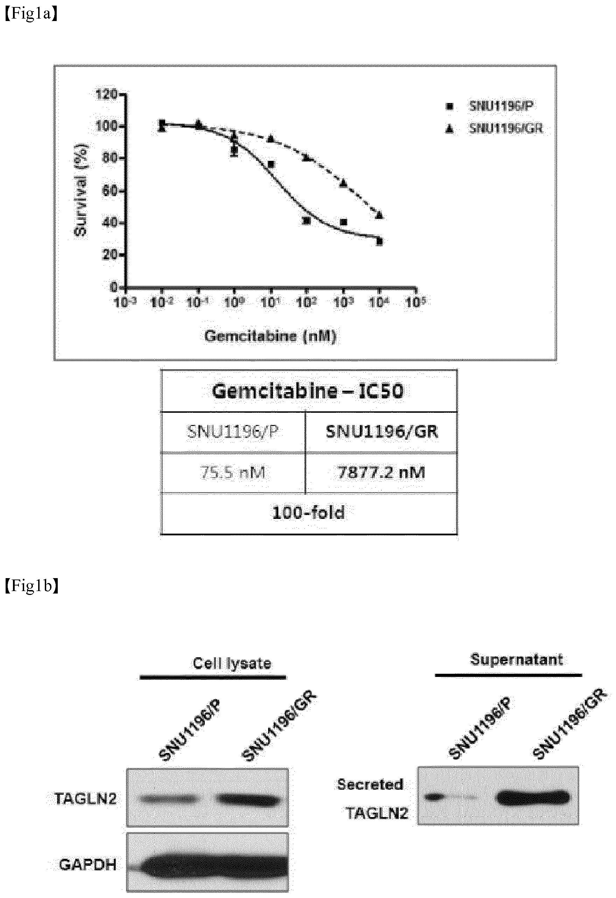

As a parent cell line SNU1196/P was cultured with an increasing concentration of gemcitabine for 1 year, it was confirmed that an IC₅₀ value of SNU1 196/GR cells for gemcitabine was 100 times higher than that of the parent cell line, and therefore the SNU1196/GR cells acquired gemcitabine resistance (FIG. 1a).

Afterward, TAGLN2 expression in the anticancer agent (gemcitabine)-resistant biliary tract cancer cell line SNU1196/GR was analyzed by western blotting, and therefore, as shown in FIG. 1b, TAGLN2 expression and secretion were considerably higher in the anticancer agent-resistant biliary tract cancer cells (SNU1196/GR) than in the parent cell line (SNU1196/P) (FIG. 1b). From the above result, it can be confirmed that TAGLN2 plays an important role in acquisition of anticancer agent resistance of the biliary tract cancer cells, and the anticancer agent resistance of the biliary tract cancer cells can be diagnosed by confirming a TAGLN2 expression level.

### Example 2. Analysis of expression of cancer stem cell marker CD151 in anticancer agent-resistant biliary tract cancer stem cell line

### Isolation and culturing of spheres having characteristics of cancer stem cells

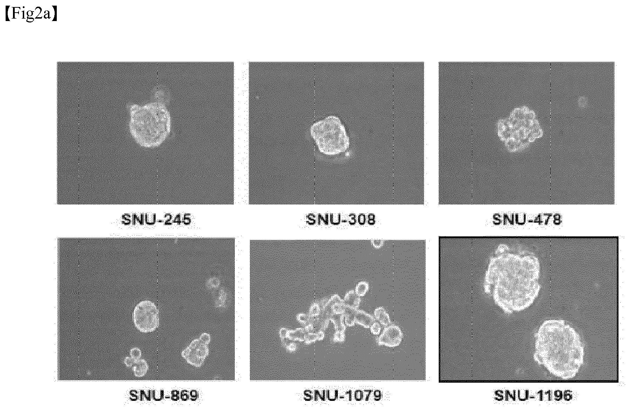

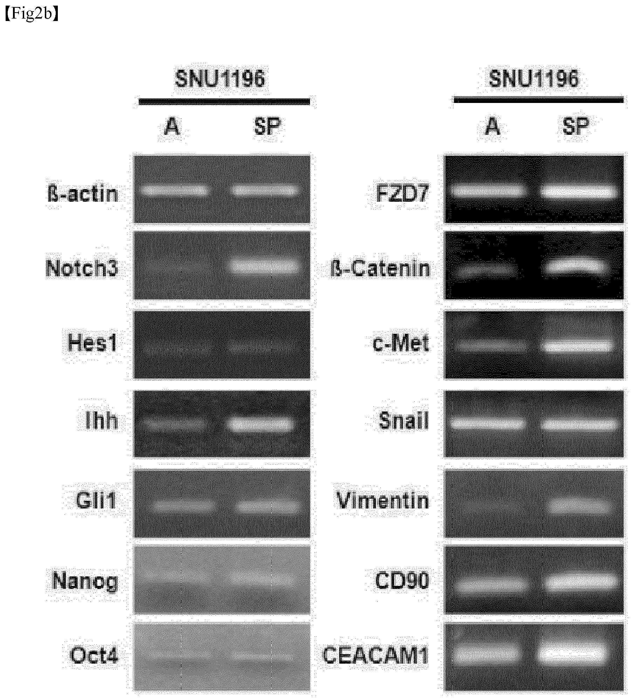

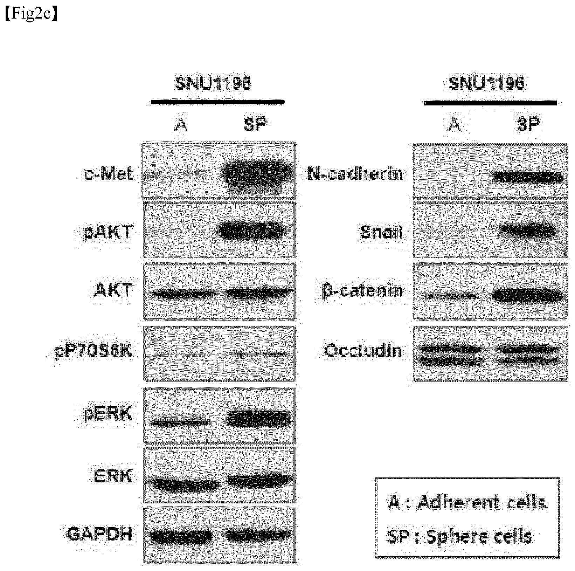

Formation of spheres exhibiting the characteristics of stem cells in a human biliary tract cancer cell line was confirmed, and a SNU1196 cell line was selected to perform a subsequent experiment (FIG. 2a). SNU1196 was selected from the sphere formation-identified biliary tract cancer cell line to isolate and culture spheres, and expression of various cancer stem cell-related genes and proteins was confirmed. It was confirmed that stem cell-related signaling systems such as Notch, Hedgehog and Wnt in sphere cells were activated, unlike in adherent cells of biliary tract cancer cell lines (FIG. 2b). In addition, it can be seen that the expression levels of c-MET as a cancer stem cell marker and downstream signals were increased, and the expression levels of N-cadherin and Snail as markers for epithelial to mesenchymal transition (EMT), which is a characteristic of cancer stem cells, were also increased (FIG. 2c).

### Confirmation of carcinogenic ability of sphere cells

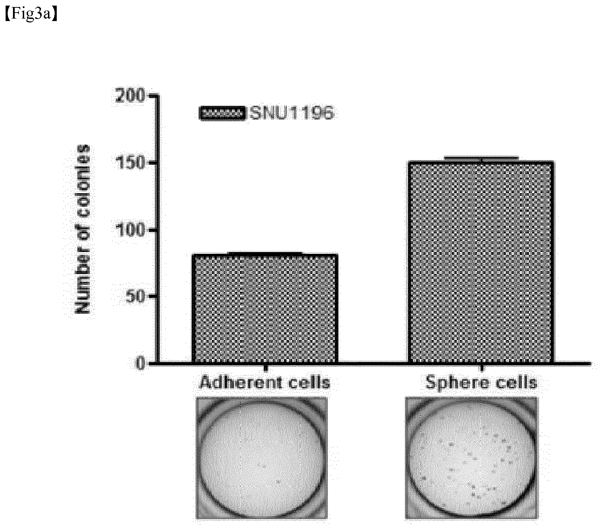

The characteristic of sphere cells cultured in SNU1196 was verified by clonogenic assay. Compared with adherent cells, it was confirmed that the sphere cells formed more colonies and greatly exhibited the most important characteristics of the stem cells, such as self-renewal and anchorage-independent growth abilities (FIG. 3a).

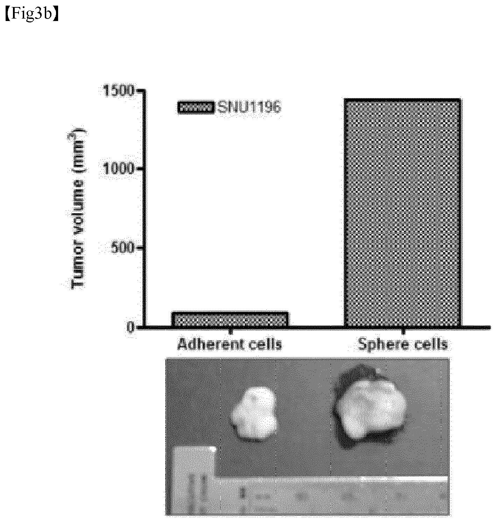

To confirm a carcinogenic ability of sphere cells of the human biliary tract cancer cell line SNU1196, 100 adherent cells as a control or sphere cells were subcutaneously injected into both sides of a nude mouse and then monitored for 12 weeks. Greater carcinogenic abilities were exhibited in a group into which the sphere cells exhibiting the characteristics of cancer stem cells were injected than in a group into which the adherent cells were injected, and therefore, it was confirmed that the sphere cells were more rapidly grown, and histologically, due to severe internal necrosis, more malignance was exhibited (FIG. 3b).

Based on the results in which the cancer stem cell-related genes and markers were overexpressed and carcinogenic ability was higher in the sphere cells formed form the biliary tract cancer cell line, the sphere cells were considered to include a large amount of cancer stem cells, and it was determined that a biliary tract cancer stem cell-specific marker can be predicted by applying a biomarker specifically expressed in the spheres.

### Analysis of CD151 expression in biliary tract cancer stem cells, cancer cell line, and human biliary tract cancer tissue

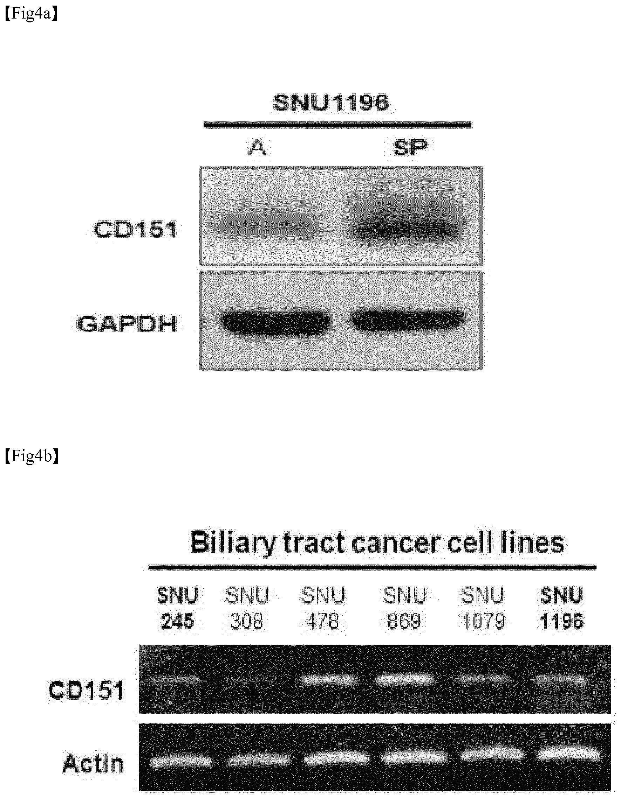

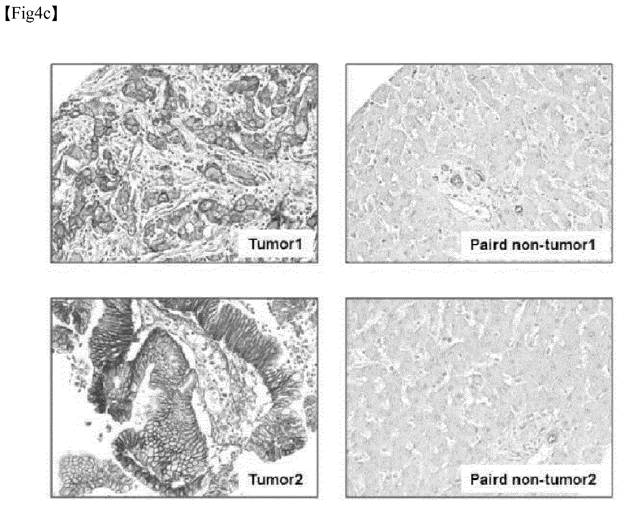

It was confirmed by western blotting that CD151 was overexpressed in the sphere cells having the characteristics of cancer stem cells (FIG. 4a). This can show that CD151 is a potential biliary tract cancer stem cell-related marker. In addition, CD151 was overexpressed in the human biliary tract cancer cell line (FIG. 4b), and it was verified that the expression of CD151 was considerably expressed in normal tissue but highly expressed in biliary tract cancer tissue by confirming CD151 expression in human normal biliary tract epithelial tissue and biliary tract cancer tissue (FIG. 4c).

### Cell isolation using antibody against biliary tract cancer stem cell-related marker, CD151, and confirmation of characteristics of cancer stem cells of isolated cells

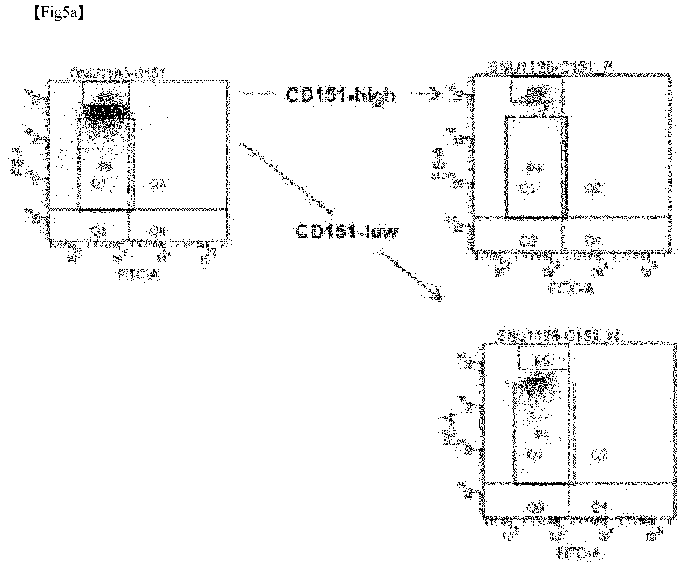

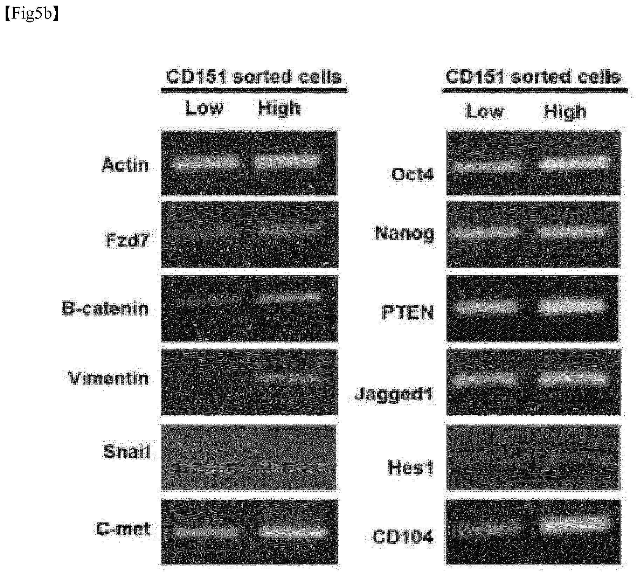

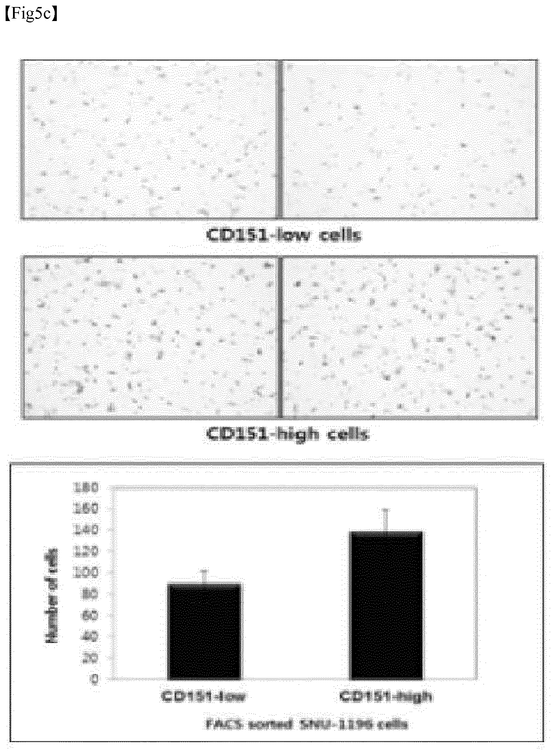

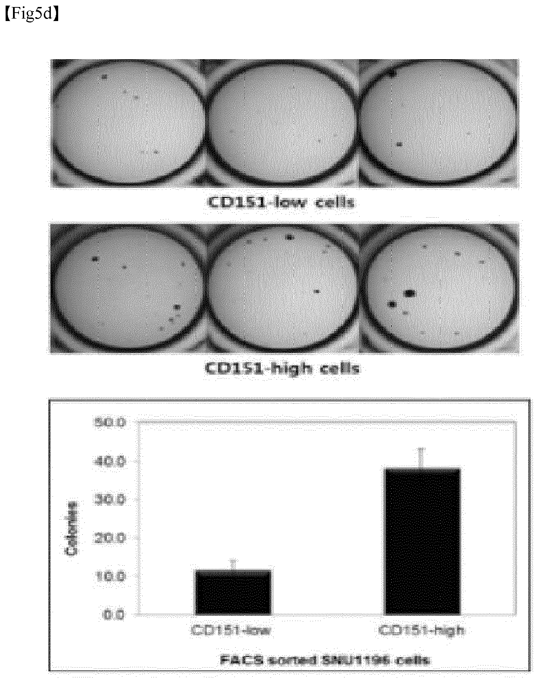

To verify the relationship between the biliary tract cancer stem cell-related marker CD151 and cancer stem cells, a biliary tract cancer cell line, that is, SNU11 96 cells, were stained using an anti-CD151 antibody by indirect FACS staining, and then isolated (FIG. 5a). Among the cells isolated by an anti-CD151 antibody, it was confirmed that OCT4 exhibiting multipotency of stem cells in cells highly expressing CD151, beta-catenin involved in maintenance and regeneration of cancer stem cells, a cancer stem cell marker, that is, c-MET, and vimentin and CD104 genes associated with cancer metastasis, which is one of various characteristics of cancer stem cells, were also increased (FIG. 5b). In addition, according to the results of cancer metastasis cell analysis and clonogenic assay to confirm the most important characteristics of the cancer stem cells such as self-renewal and anchorage-independent growth, it was verified that the cells in which CD151 was highly expressed exhibited high cancer metastatic and self-renewal abilities (FIGS. 5c and 5d).

### Analysis of change in characteristics of cancer stem cells according to inhibition of CD151 expression

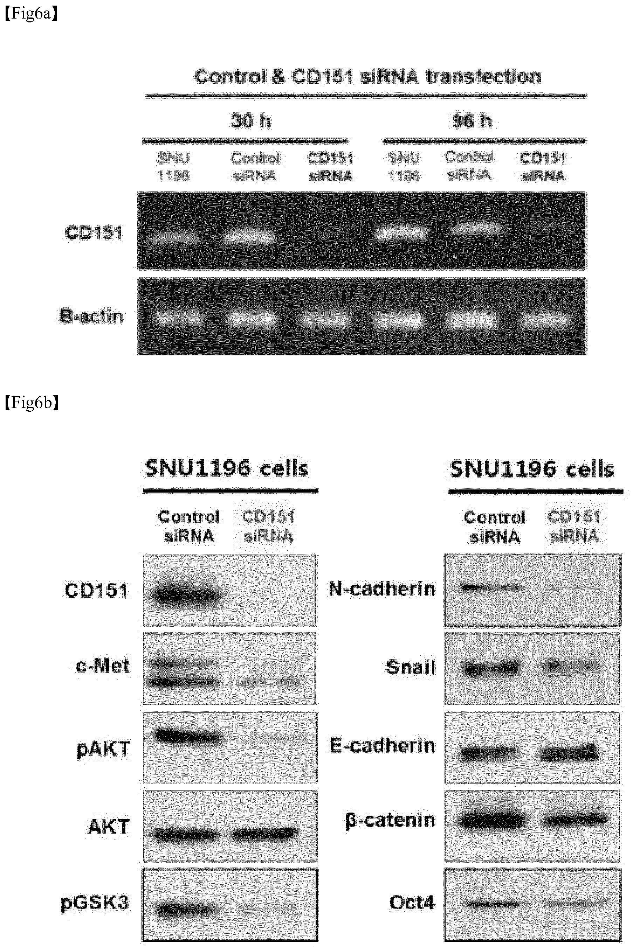

When an SNU1196 cell line was transfected with CD151 siRNA, it was confirmed that CD151 mRNA expression was apparently reduced for 30 to 96 hours (FIG. 6a).

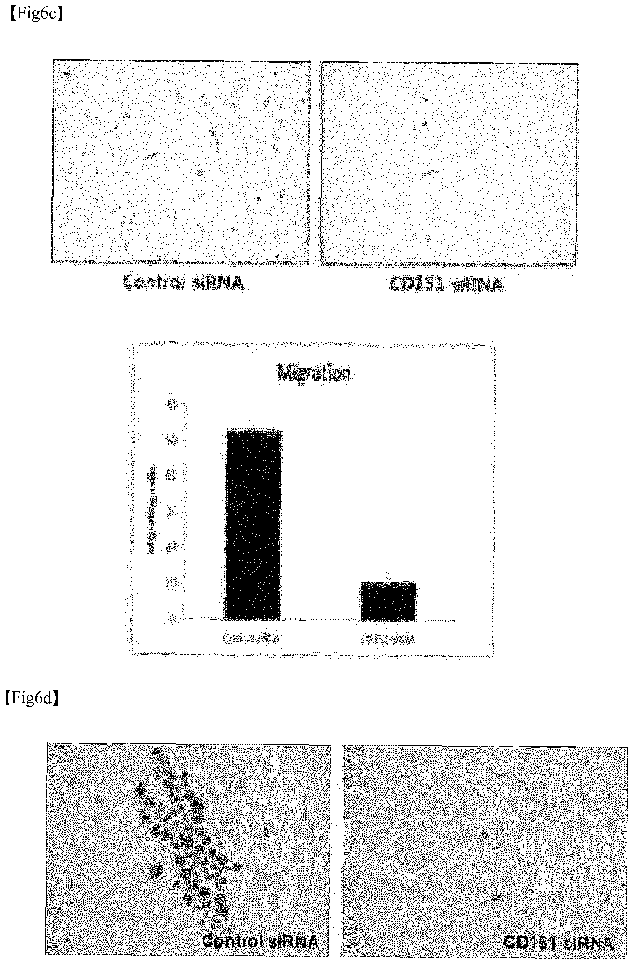

A change in expression of various proteins after 72 hours of the CD151 siRNA transfection was confirmed by western blotting, and therefore it was confirmed that expression of c-MET, known as a cancer stem cell marker, and of downstream signals such as phosphorylated AKT and GSK3 was reduced while CD151 expression was inhibited, and that beta-catenin of a cancer stem cell-related Wnt signaling system, OCT4 exhibiting multipotent ability of stem cells, and N-cadherin and Snail, which are cancer metastasis-related genes, were reduced (FIG. 6b). In addition, when CD151 expression was inhibited, inhibition of cancer metastatic ability (FIG. 6c) and inhibition of self-renewal ability (FIG. 6d) were shown, and it can be considered that CD151 is a biliary tract cancer stem cell-related marker and a therapeutic target.

### Confirmation of reduction in expression of cancer stem cell marker CD151 in anticancer agent-resistant biliary tract cancer cell line

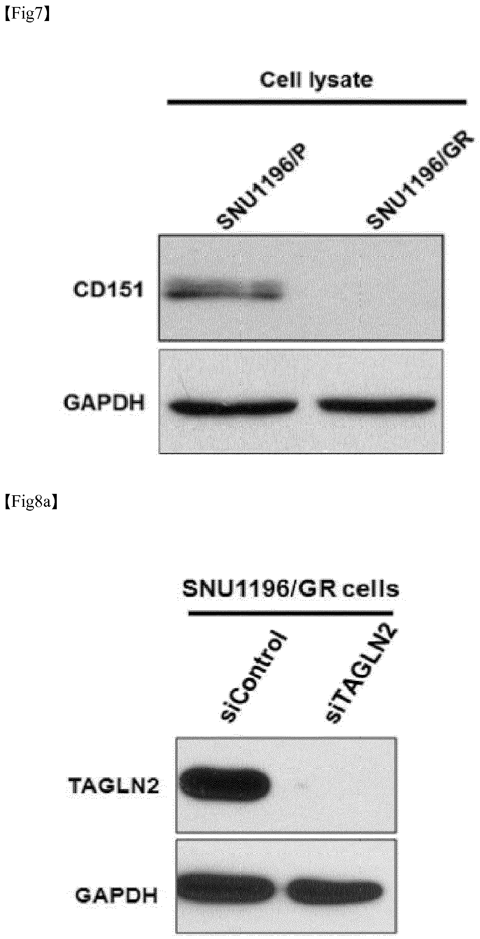

From the results of FIGS. 2 to 6, it was verified that CD151 is a biliary tract cancer stem cell marker. However, an expression level of CD151 was reduced even in the anticancer agent-resistant biliary tract cancer cell line, unlike that of TAGLN2 (FIG. 7).

### Example 3. Confirmation of overcoming anticancer agent resistance by inhibiting TAGLN2 expression in anticancer agent-resistant biliary tract cancer cell line

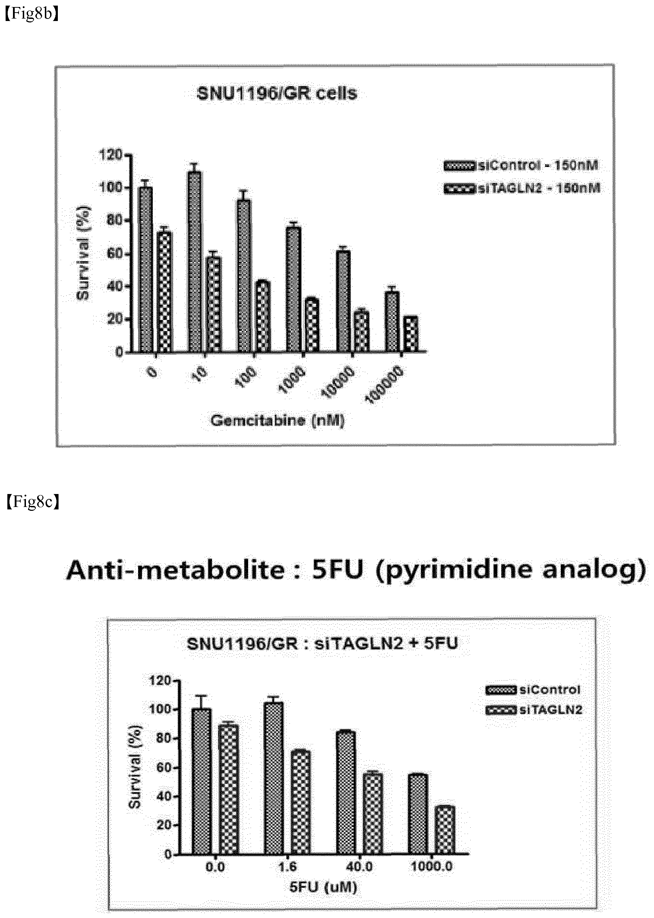

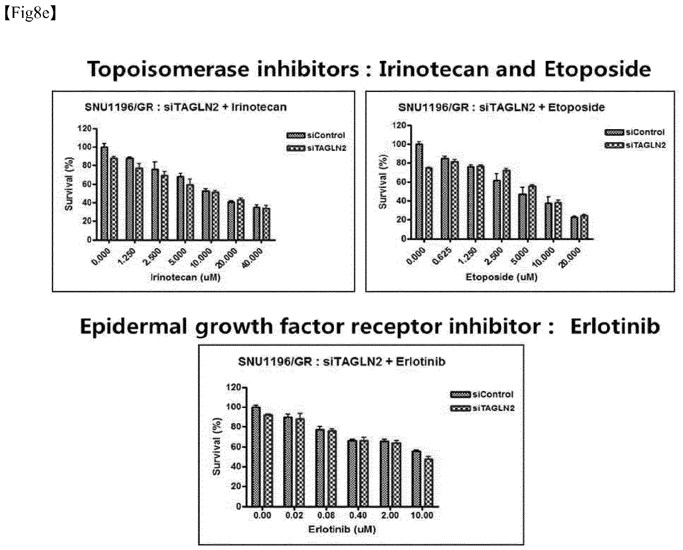

To determine whether anticancer agent resistance was overcome by inhibiting TAGLN2 expression in an anticancer agent-resistant biliary tract cancer cell line, western blotting and MTT analysis were performed. It was confirmed by western botting that TAGLN2 expression was considerably inhibited by transfection using 150 nM TAGLN2 siRNA in anticancer agent-resistant biliary tract cancer cells (SNU1196/GR) (FIG. 8a), and it was shown that co-treatment with gemcitabine (FIG. 8b) increased anticancer agent susceptibility by 43% even at a concentration of 10 nM, which was lower than the IC₅₀ concentration of a parent cell line.

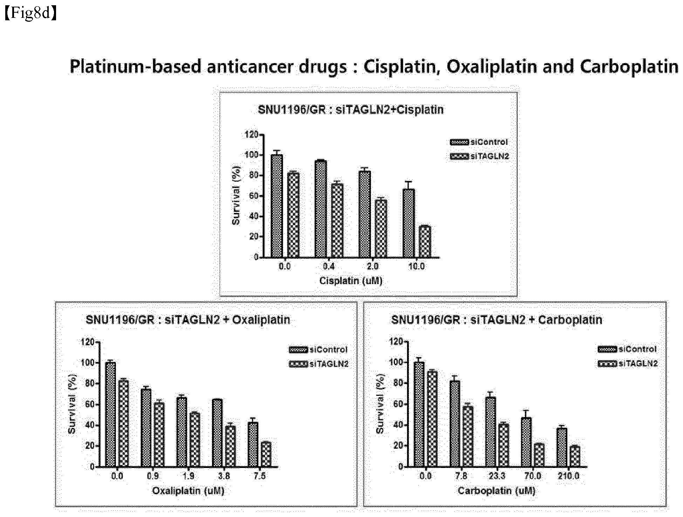

In addition, when TAGLN2 expression was inhibited in a biliary tract cancer anticancer agent-resistant cell line SNU1196/G, susceptibilities to an anti-metabolite-like anticancer agent such as 5-FU (FIG. 8c) and platinum-like anticancer agents such as cisplatin, oxaliplatin, and carboplatin (FIG. 8d) were increased.

Meanwhile, inhibiting TAGLN2 expression did not cause changes in susceptibility to topoisomerase inhibitors such as irinotecan and etoposide, and an EGFR inhibitor such as erlotinib.

From the experimental results of the present invention, it was confirmed that TAGLN2 was overexpressed in the anticancer agent-resistant biliary tract cancer cell line, and susceptibilities to 5-FU and platinum-like anticancer agents (cisplatin, oxaliplatin, and carboplatin) as well as gemcitabine can be enhanced by siTAGLN2 transfection targeting TAGLN2.

From the above-described results, it was identified that TAGLN2 is a novel therapeutic target for overcoming the resistance to an anticancer agent for biliary tract cancer.

As above, specific parts of the present invention were described in detail. It is apparent to those of ordinary skill in the art that such specific descriptions are merely specific embodiments, and the scope of the present invention is not limited thereto. Therefore, the substantial scope of the present invention is to be defined by the accompanying claims and equivalents thereof.

## Claims

1. A kit for diagnosing resistance to an anticancer agent for biliary tract cancer, comprising:
an antibody, aptamer, oligopeptide or peptide nucleic acid (PNA) binding to transgelin-2 (TAGLN2) protein, or a primer or probe binding to a nucleic acid molecule encoding the protein.

2. A kit for detecting a marker for resistance to an anticancer agent for biliary tract cancer, comprising:
an antibody, aptamer, oligopeptide or peptide nucleic acid (PNA) binding to transgelin-2 (TAGLN2) protein, or a primer or probe binding to a nucleic acid molecule encoding the protein.

3. The kit of claim 1 or 2, wherein the kit is an immunoassay kit.

4. The kit of claim 3, wherein the immunoassay kit is a Luminex analysis kit, a protein microarray kit or an ELISA kit.

5. The kit of claim 1 or 2, wherein the kit is a microarray or gene amplification kit.

6. A method for detecting a marker for resistance to an anticancer agent for biliary tract cancer, comprising:
detecting transgelin-2 (TAGLN2) protein contained in a biological sample or a nucleic acid molecule encoding the protein in order to obtain information necessary for diagnosis of resistance to an anticancer agent for biliary tract cancer.

7. The method of claim 6, wherein the method is performed by an antigen-antibody reaction.

8. The method of claim 6, wherein the method is performed by gene amplification.

9. The method of claim 6, wherein the biological sample is selected from the group consisting of biliary tract cancer cells or a culture thereof, biliary tract cancer tissue, blood, serum, and plasma.

10. A pharmaceutical composition for inhibiting resistance to an anticancer agent for biliary tract cancer, comprising:
an activation inhibitor or expression inhibitor for transgelin-2 (TAGLN2) as an active ingredient.

11. The composition of claim 10, wherein the anticancer agent is selected from the group consisting of gemcitabine, 5-fluorourasil (5-FU), oxaliplatin, carboplatin, and cisplatin.

12. The composition of claim 10, wherein the activation inhibitor for TAGLN2 is selected from the group consisting of an antibody specifically binding to TAGLN2, and a fragment, a compound, a peptide, a peptide mimetic and an aptamer, which bind to an antigen thereof.

13. The composition of claim 10, wherein the TAGLN2 expression inhibitor is selected from the group consisting of an antisense oligonucleotide complementarily binding to mRNA of a TAGLN2 gene or TAGLN2 expression-promoting gene, small interfering RNA (siRNA), small hairpin RNA (shRNA), and ribozyme.

14. A method for inhibiting resistance to an anticancer agent for biliary tract cancer, comprising:
administering an activation inhibitor or expression inhibitor for transgelin-2 (TAGLN2) to a subject.

15. The method of claim 14, wherein the anticancer agent is selected from the group consisting of gemcitabine, 5-fluorourasil (5-FU), oxaliplatin, carboplatin, and cisplatin.

16. The method of claim 15, wherein the TAGLN2 activation inhibitor is selected from the group consisting of an antibody binding to TAGLN2, and a fragment, a compound, a peptide, a peptide mimetic and an aptamer, which bind to an antigen thereof.

17. The method of claim 14, wherein the TAGLN2 expression inhibitor is selected from the group consisting of an antisense oligonucleotide complementarily binding to mRNA of a TAGLN2 gene or of a TAGLN2 expression-promoting gene, small interfering RNA (siRNA), small hairpin RNA (shRNA), and ribozyme.
